# EUROPEAN PATENT APPLICATION

(11) **EP 4 830 636 A1**
(43) Date of publication of application: **28.10.2026**
(21) Application number: 24906125.0
(22) Date of filing: 05.12.2024
(51) Int. Cl.: A61B 6/03

(54) **RADIATION IMAGING DEVICE, CT IMAGING DEVICE, AND IMAGING METHOD**

(30) Priority: 21.12.2023 CN 202311773750
(71) Applicant: Nuctech Company Limited, TongFang Building Shuangqinglu Haidian District Beijing 100084 (CN); Tsinghua University, Beijing 100084 (CN)
(72) Inventor: CHEN, Zhiqiang, Beijing 100084 (CN); ZHANG, Li, Beijing 100084 (CN); LI, Xinbin, Beijing 100084 (CN); WANG, Zhentian, Beijing 100084 (CN); HUANG, Qingping, Beijing 100084 (CN); SUN, Yunda, Beijing 100084 (CN); SHEN, Le, Beijing 100084 (CN); FENG, Bo, Beijing 100084 (CN)
(74) Representative: Cabinet Beau de Loménie
(86) International application number: PCT/CN2024/137108
(87) International publication number: WO 2025/130641

(57) **Abstract**

The present disclosure provides a radiation imaging apparatus. The apparatus includes: a radiation source configured to emit a radiation beam; a detector configured to receive the radiation beam from the radiation source, where the radiation source and the detector define an inspection channel; and one or more gratings arranged in the inspection channel and between the radiation source and the detector. The one or more gratings have an arc surface, or a groove-shaped surface including a plurality of angled surface segments. The arc surface or the groove-shaped surface faces the radiation source.

## Description

This application claims priority to Chinese Patent Application No. 202311773750.8 filed on December 21, 2023, the entire contents of which are incorporated herein by reference.

### TECHNICAL FIELD

The present disclosure relates to a field of detection technology. Specifically, the present disclosure relates to a radiation imaging apparatus and method, and a CT imaging apparatus and method, and in particular to a grating-based radiation imaging apparatus and method, and a grating-based CT imaging apparatus and method.

### BACKGROUND

Related radiation imaging apparatuses are usually implemented based on the existing X-ray absorption imaging. The core components of such apparatuses include an X-ray source and a detector, which may obtain absorption images reflecting attenuation characteristics of X-rays passing through substances. Such absorption images exhibit strong discriminating capability for high-density substances having strong X-ray absorption capability, and provide excellent imaging effects for substances such as metals, bones, and the like. Therefore, absorption images have been widely used in security inspection fields for identifying contraband and in medical fields for diagnosing bone diseases.

However, for weakly absorbing substances composed of light elements (such as carbon, hydrogen, oxygen, nitrogen, etc.), the corresponding degree of attenuation is relatively low, resulting in weak signals in the absorption image, thus posing significant limitations. For example, in the field of security inspection, it is difficult to observe contraband such as drugs obscured by metal, and in the medical field, it is difficult to observe pathological tissues in the lungs. On the other hand, signal differences between different weakly absorbing substances are relatively small, making them difficult to distinguish from each other. These issues may lead to misidentification of target objects, such as missed detections of drug contraband during security inspections or failure to clearly visualize lesion information in the lungs during medical diagnosis. In summary, in order to improve the effectiveness of security inspection and medical diagnosis, new technologies and new products that help enhance the image discriminating capability for weakly absorbing substances may be explored.

### SUMMARY

According to an aspect of the present disclosure, a radiation imaging apparatus is provided, including: a radiation source configured to emit a radiation beam; a detector configured to receive the radiation beam from the radiation source, where the radiation source and the detector define an inspection channel; and one or more gratings arranged in the inspection channel and between the radiation source and the detector; where the one or more gratings have one of an arc surface structure, a groove-shaped surface structure, or an L-shaped surface structure, the detector is in one of an arc shape, a groove shape, or an L shape to extend parallel to the arc surface structure, the groove-shaped surface structure, or the L-shaped surface structure of the one or more gratings, and the arc surface, the groove-shaped surface, or the L-shaped surface is arranged to face the radiation source to receive the radiation beam emitted by the radiation source.

In an embodiment, the one or more gratings include periodic substructures configured to diffract rays to generate an interference fringe.

In an embodiment, the one or more gratings include a spherical grating.

In an embodiment, for the one or more gratings having the groove-shaped surface structure or the L-shaped surface structure, an extension direction of each of the periodic substructures forms a non-zero angle with an extension direction of the inspection channel.

In an embodiment, the extension direction of each of the periodic substructures is perpendicular to the extension direction of the inspection channel.

In an embodiment, the detector and the one or more gratings each have an integral structure.

In an embodiment, the detector and the one or more gratings each include a plurality of surface segments forming an arc surface, or include a plurality of angled surface segments forming a groove-shaped surface or an L-shaped surface.

In an embodiment, the plurality of surface segments of the arc surface, or the plurality of angled surface segments of the groove-shaped surface or the L-shaped surface, abut against each other or are spaced apart from each other.

In an embodiment, the one or more gratings include: a first grating arranged adjacent to and parallel to the radiation source and spaced apart from the radiation source by a first distance; and/or a second grating arranged adjacent to and parallel to the radiation source and spaced apart from the radiation source by a second distance different from the first distance; or a second grating arranged adjacent to and parallel to the detector and spaced apart from the detector by a third distance; and/or a third grating arranged adjacent to and parallel to the detector and spaced apart from the detector by a fourth distance different from the third distance.

In an embodiment, the radiation imaging apparatus includes the first grating, the second grating, and the third grating, where the first grating, the second grating, and the third grating are parallel to each other.

In an embodiment, the radiation imaging apparatus is configured to generate, based on signals detected by the detector, at least one of an absorption image, a phase image, or a small-angle scattering image related to at least a partial region of an object under inspection in the inspection channel.

In an embodiment, the radiation source is a dual-energy radiation source.

According to another aspect of the present disclosure, a CT imaging apparatus is provided, including: a slip ring configured to be rotatable; and the radiation imaging apparatus according to the present disclosure, where the radiation imaging apparatus is arranged on the slip ring to rotate with the slip ring; where the radiation source, the detector, and the one or more gratings are arranged on the slip ring, and the inspection channel passes through the slip ring.

According to another aspect of the present disclosure, a radiation imaging method is provided, including: irradiating an object under inspection located in an inspection channel with a radiation beam by using the radiation imaging apparatus according to the present disclosure; and collecting signals using a detector so as to generate, based on the signals detected by the detector, at least one of an absorption image, a phase image, or a small-angle scattering image related to at least a partial region of the object under inspection in the inspection channel.

According to another aspect of the present disclosure, a CT imaging method is provided, including: irradiating an object under inspection located in an inspection channel with a radiation beam by using the CT imaging apparatus according to the present disclosure; and collecting signals using a detector so as to generate, based on the signals detected by the detector, at least one of a three-dimensional absorption image, a three-dimensional phase image, or a three-dimensional small-angle scattering image related to at least a partial region of the object under inspection in the inspection channel.

It may be understood that the content described in this section is not intended to identify key or essential features of embodiments of the present disclosure, nor is it intended to limit the scope of the present disclosure. Other features of the present disclosure will become readily understandable through the following description.

### BRIEF DESCRIPTION OF THE DRAWINGS

The accompanying drawings are provided for better understanding of the present solution and do not constitute any limitations to the present disclosure, in which:
FIG. 1 shows various grating imaging arrangements in (a) to (d);
FIG. 2 to FIG. 5 show schematic diagrams of different embodiments of a radiation imaging apparatus according to embodiments of the present disclosure;
FIG. 6 shows a schematic diagram of a periodic substructure of a grating according to an embodiment of the present disclosure;
FIG. 7 to FIG. 10 show schematic diagrams of different embodiments of a radiation imaging apparatus according to embodiments of the present disclosure;
FIG. 11 shows, in (a) to (c), schematic diagrams of different embodiments of a CT imaging apparatus according to embodiments of the present disclosure;
FIG. 12 shows a perspective view of a radiation imaging apparatus according to an embodiment of the present disclosure in which a grating has an arc surface structure;
FIG. 13 shows two-dimensional images for absorption T (a), phase Φ (b), and small-angle scattering D (c), as well as corresponding XY-plane slice images of three-dimensional images for linear attenuation coefficient *µ* (d), refractive index decrement *δ* (e), and linear diffusion coefficient ζ (f) reconstructed using an FBP algorithm from 360 projections acquired over one rotation; and
FIG. 14 shows stained histological microscopic images (a1 to a6) of human breast specimens 1 to 6, as well as corresponding XZ-plane slice images of reconstructed three-dimensional images for linear attenuation coefficient *µ* (b1 to b6) and XZ-plane slice images of reconstructed three-dimensional images for refractive index decrement *δ* (c1 to c6).

### DETAILED DESCRIPTION OF EMBODIMENTS

To more clearly describe the objectives, technical solutions, and advantages of the present disclosure, embodiments of the present disclosure will be described in detail below with reference to the accompanying drawings. It may be understood that the following description of embodiments is intended to explain and illustrate the general concept of the present disclosure and may not be construed as limiting the present disclosure. In the specification and the accompanying drawings, the same or similar reference numerals denote the same or similar components or members. For purposes of clarity, the drawings are not necessarily drawn to scale, and certain well-known components and structures may be omitted from the drawings.

Unless otherwise defined, technical or scientific terms used herein shall have the meanings commonly understood by a person having ordinary skill in the art to which the present disclosure pertains. Terms such as "first", "second", and similar expressions used herein do not indicate any order, quantity, or importance, but are merely used to distinguish different components. Terms "a" and "an" do not exclude the presence of multiple. Terms such as "comprising", "including", or similar expressions indicate that the element or item preceding the word encompasses the element or item listed thereafter as well as equivalents thereof, but does not exclude additional elements or items. Terms such as "connected", "coupled", or similar expressions are not limited to physical or mechanical connections and may include electrical connections, whether direct or indirect. Terms such as "upper", "lower", "left", "right", "top", "bottom", and the like are used only to indicate relative positional relationships, and such relative positional relationships may change when the absolute position of the described object changes. When an element such as a layer, film, region, or substrate is described as being arranged "on" or "under" another element, the element may be directly on or under the other element, or one or more intermediate elements may be present therebetween.

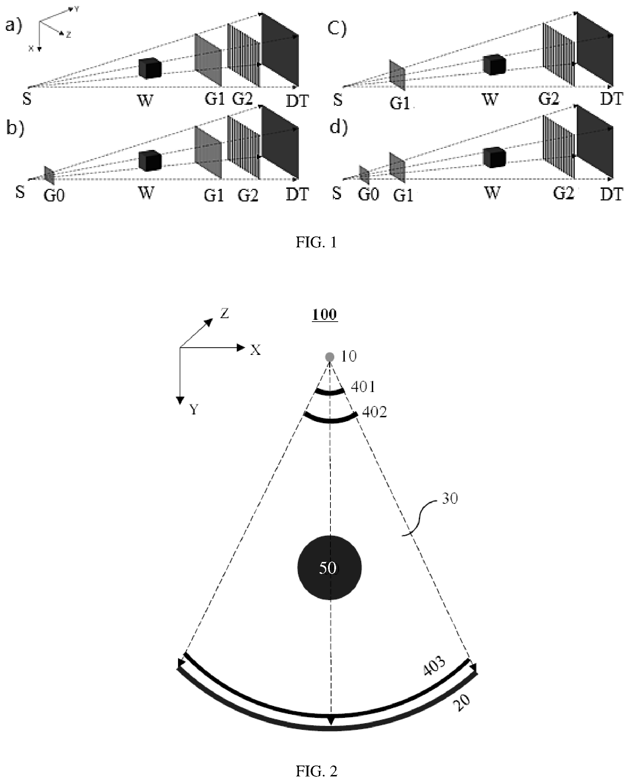

In (a), (b), (c), and (d) of FIG. 1, various grating imaging arrangements are shown. G0, G1, and G2 represent three gratings, which are respectively arranged between a source S and an object under inspection W or between the object under inspection W and a detector DT as shown in (a), (b), (c), and (d) of FIG. 1.

In grating imaging technology, both the wave nature and the particle nature of X-rays may be utilized simultaneously to acquire multi-characteristic information including three types of information, namely absorption information, phase information, and small-angle scattering information, where the absorption information and the phase information respectively correspond to an imaginary part *β* and a real part *δ* of a complex refractive index *n*. $n = 1 - δ + iβ$ (1) where a linear attenuation coefficient µ has a linear relationship with the imaginary part β (λ represents a wavelength of the X-rays). $μ = \frac{4 πβ}{λ}$ (2)

The three types of characteristic information of absorption *T*, phase Φ, and small-angle scattering D may be respectively regarded as integrals of the linear attenuation coefficient µ, the refractive index decrement *δ*, and the linear diffusion coefficient *ζ* (where *L* represents a propagation path of X-rays, and *c*_{Φ}, and *c_{D}* are both constant values related to the imaging system). $\left\{\begin{matrix}T = exp \left(-{\int }_{L} μ \left(\vec{r}\right) d \vec{r}\right) \\ Φ = {c}_{Φ} ⋅ \frac{\partial }{\partial x} \left({\int }_{L} δ \left(\vec{r}\right) d \vec{r}\right) \\ D = {c}_{D} ⋅ exp \left(-{\int }_{L} ζ \left(\vec{r}\right) d \vec{r}\right)\end{matrix}\right.$ (3)

Considering that X-rays generally exhibit an energy spectrum distribution, and assuming that a normalized energy spectrum distribution is represented by S(E), Equation (3) may be expressed as: $\left\{\begin{matrix}T = {\int }_{{E}_{0}}^{{E}_{1}} S \left(E\right) ⋅ exp \left(-{\int }_{L} μ \left(\vec{r}, E\right) d \vec{r}\right) dE \\ Φ = {c}_{Φ} ⋅ {\int }_{{E}_{0}}^{{E}_{1}} S \left(E\right) ⋅ \frac{\partial }{\partial x} \left({\int }_{L} δ \left(\vec{r}, E\right) d \vec{r}\right) dE \\ D = {c}_{D} ⋅ {\int }_{{E}_{0}}^{{E}_{1}} S \left(E\right) ⋅ exp \left(-{\int }_{L} ζ \left(\vec{r}, E\right) d \vec{r}\right) dE\end{matrix}\right.$ (4)

In (a) of FIG. 1, the grating G1 may function to form a self-imaging fringe pattern along a downstream optical path. When X-rays interact with a substance, the self-imaging fringe pattern may undergo local distortion and deformation. Such deformation occurs on a micrometer scale or even a sub-micrometer scale, which is beyond the resolution that may be achieved by ordinary detectors. Therefore, grating G2 amplifies the signal. By observing variations in the intensity of the detector signal, three characteristic images of absorption, phase, and small-angle scattering may be extracted. When a focal spot of the X-ray source is sufficiently small (e.g., in a synchrotron radiation source or a micro-focus X-ray source), only gratings G1 and G2 are required, as shown in (a) and (c) of FIG. 1.

When the focal spot of the X-ray source has a large size (e.g., in a conventional X-ray source), the resulting blurring effect may deteriorate the imaging effect. Therefore, a grating G0 may be added downstream of the X-ray source. Due to the presence of grating G0, the radiation beams emitted by the X-ray source may be converted into a series of partially coherent micrometer-scale linear radiation beams, as shown in (b) and (d) of FIG. 1.

An object under inspection W may be positioned between grating G0 and grating G1 (as shown in (b) of FIG. 1), or between grating G1 and grating G2 (as shown in (c) and (d) of FIG. 1).

When the object under inspection W is positioned between gratings G0 and G1, as shown in (b) of FIG. 1, in this configuration, stronger phase signals and small-angle scattering signals may be obtained, resulting in higher imaging contrast and sensitivity. However, this configuration also has certain disadvantages, that is, grating G1 has a large area and incurs higher cost. In addition, X-rays transmitted through the object under inspection W may be blocked by gratings G1 and G2 before being received by the detector, resulting in low X-ray utilization efficiency.

When the object under inspection W is positioned between gratings G1 and G2, as shown in (c) and (d) of FIG. 1, in this configuration, grating G1 is located close to the light source and may have a small area and low cost. Furthermore, X-rays transmitted through the object under inspection W are blocked only by grating G2, resulting in higher X-ray utilization efficiency. However, the phase signals and small-angle scattering signals obtained in this configuration are somewhat weaker. Nevertheless, for certain strongly scattering substances, this is less likely to cause detector signal saturation.

The absorption image (represented by T above) reflects attenuation characteristics of the substance with respect to X-rays, corresponding to the imaginary part *β* of the complex refractive index of the substance for X-rays (complex refractive index *n* = 1 - *δ* + *iβ*). The phase image (represented by Φ above) reflects refraction effects of the substance on X-rays, corresponding to the real part *δ* of the complex refractive index of the substance for X-rays. For weakly absorbing substances, the phase image advantageously provides higher contrast than the absorption image, and *δ* may be higher than *β* by 2 to 3 orders of magnitude. Accordingly, weakly absorbing substances may be displayed more clearly through the phase image. The small-angle scattering image (represented by *D* above) reflects a mesoscopic structural information of the substance and is highly sensitive to differences at the micrometer scale or sub-micrometer scale, such as porosity and multi-fiber structures. Accordingly, the small-angle scattering image may break through the resolution limitations of absorption imaging. The phase image and the small-angle scattering image are valuable supplements to the absorption image, which may effectively improve the overall imaging effect and resolving capability for the object under inspection, and enhance the adaptability and accuracy of the inspection process for different substances.

The present disclosure provides a radiation imaging apparatus and method, and a CT imaging apparatus and method. By introducing grating elements, the radiation imaging apparatus is capable of additionally obtaining phase images and small-angle scattering images as supplements to absorption images, thereby improving the imaging discrimination capability with respect to weakly absorbing substances. Accordingly, performance metrics of the imaging system may be improved, which is conducive to further improving the detection rate of contraband in security inspection scenarios and further enhancing disease imaging diagnosis in medical scenarios.

FIG. 2 to FIG. 4 show schematic diagrams of different embodiments of a radiation imaging apparatus 100 according to embodiments of the present disclosure. As shown in FIG. 2 to FIG. 4, the radiation imaging apparatus 100 may include a radiation source 10 and a detector 20. According to embodiments of the present disclosure, the radiation source 10 is configured to emit a radiation beam or radiation rays. By way of example, the radiation source 10 may be an X-ray source configured to emit X-rays. However, embodiments of the present disclosure are not limited thereto. The radiation source 10 and the detector 20 may be arranged opposite to each other in the Y direction, such that the detector 20 may receive the radiation beam or radiation rays emitted from the radiation source 10. The detector 20 may extend in the Z direction and cooperate with the radiation source 10 to define an inspection channel 30 extending in the Z direction. When the radiation imaging apparatus 100 is used to perform inspection, an object under inspection 50 may be placed in the inspection channel 30. Accordingly, the detector 20 may generate a detector signal based on the received radiation beam or radiation rays, thereby forming an inspection image related to at least a partial region of the object under inspection 50.

According to embodiments of the present disclosure, the one or more gratings are arranged in the inspection channel 30 and between the radiation source 10 and the detector 20. By way of example, FIG. 2 to FIG. 4 show the arrangement of three gratings, i.e., a first grating 401, a second grating 402, and a third grating 403. The first grating 401 and the second grating 402 may be arranged adjacent to the radiation source 10 and may be spaced apart from the radiation source 10 by a first distance and a second distance, respectively. For example, the second distance may be greater than the first distance. The third grating 403 may be arranged adjacent to the detector 20. However, embodiments of the present disclosure are not limited thereto. The gratings may be arranged in various manners, such as those described above with reference to FIG. 1. For example, as shown in (a) and (c) of FIG. 1, only two gratings may be arranged between the radiation source 10 and the detector 20. Alternatively, as shown in (a) and (b) of FIG. 1, two gratings may be arranged between the object under inspection 50 and the detector 20. That is, the second grating 402 and the third grating 403 may be arranged adjacent to the detector 20 and may be spaced apart from the detector 20 by a third distance and a fourth distance, respectively. For example, the third distance may be greater than the fourth distance. It may be noted that the arrangement positions and the number of gratings may be appropriately adjusted according to practical requirements. For ease of understanding, the description herein will be provided with reference to the grating arrangements shown in FIG. 2 to FIG. 4. However, it may be understood that the content described herein may apply to other grating arrangements. In other embodiments of the present aspect, only the grating 401 may be provided, only the grating 402 may be provided, only the grating 403 may be provided, only the grating 401 and the grating 402 may be provided, only the grating 401 and the grating 403 may be provided, or only the grating 402 and the grating 403 may be provided. According to the present disclosure, any grating and any combination of gratings may be employed in different embodiments.

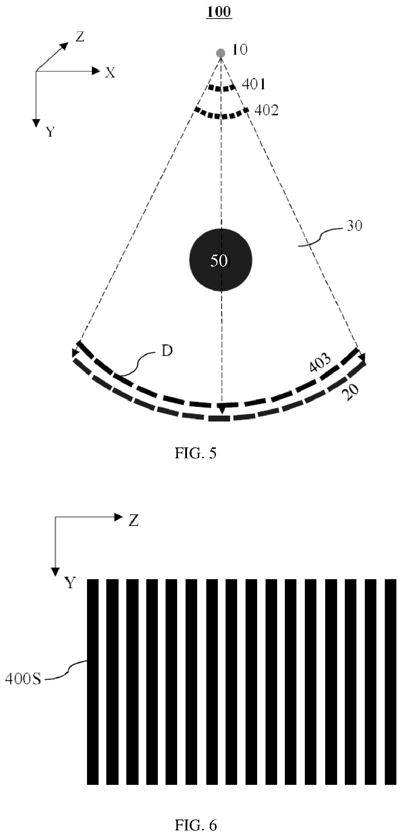

Herein, a grating may refer to an optical device that includes a periodic substructure used to diffract the radiation beams or radiation rays to generate one or more interference fringes. By way of example, the "periodic substructure" of a grating may be understood as grating lines, such as grating lines 400S shown in FIG. 6. In an embodiment, the grating may have a slit structure through which light is diffracted to form interference fringes. In an embodiment, the grating may include a material capable of modulating electromagnetic waves or radiation into interference fringes. As described above, by utilizing the interference fringe formed by the grating, the radiation imaging apparatus 100 may not only obtain an absorption image of the object under inspection 50, but may also selectively obtain a phase image and/or a small-angle scattering image, thereby improving imaging performance metrics of the radiation imaging apparatus 100 and enhancing imaging discrimination capability with respect to the object under inspection 50. That is, the detector 20 of the radiation imaging apparatus 100 according to embodiments of the present disclosure may generate at least one of an absorption image, a phase image, or a small-angle scattering image related to at least a partial region of the object under inspection 50.

As shown in FIG. 2 to FIG. 4, the gratings may be configured to have different structures. Accordingly, the shape of the detector 20 may be configured according to the structure of the gratings, such that the detector 20 may cooperate with the gratings to generate appropriate detector signals. Furthermore, depending on different shapes of the detector 20, inspection channels 30 having different configurations may be defined.

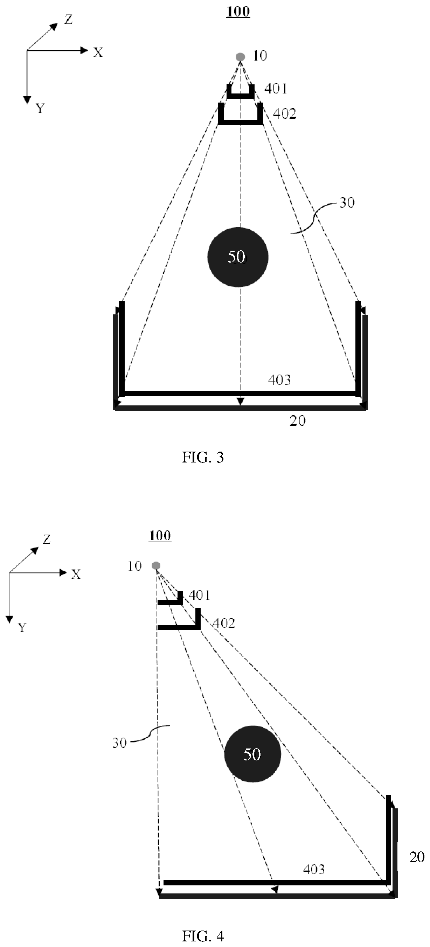

Specifically, the first grating 401, the second grating 402, and the third grating 403 may be configured to have the same structure and may be parallel to each other. By way of example, FIG. 2 illustrates that the three gratings 401 to 403 each have an arc surface structure and are arranged parallel to each other with the same curvature. FIG. 3 illustrates that the three gratings 401 to 403 each have a groove-shaped surface structure. FIG. 4 illustrates that the three gratings 401 to 403 each have an L-shaped surface structure. Further, the detector 20 may be configured to extend parallel to the structure of the gratings, thereby forming a corresponding shape. By way of example, the detector 20 in FIG. 2 is in an arc shape parallel to the arc surface structures of the gratings; the detector 20 in FIG. 3 is in a groove shape parallel to the groove-shaped surface structures of the gratings; and the detector 20 in FIG. 4 is in an L shape parallel to the L-shaped surface structures of the gratings. Accordingly, the first grating 401, the second grating 402, the third grating 403, and the detector 20 may be arranged parallel to each other, thereby cooperating with each other to generate detector signals corresponding to the radiation beam in respective regions.

As shown in FIG. 2 to FIG. 4, the arc surface structures, groove-shaped surface structures, or L-shaped surface structures of the gratings, and the arc shape, groove shape, or L shape of the detector 20, are arranged to face the radiation source 10 to receive the radiation beam emitted by the radiation source 10. Furthermore, in FIG. 2, the arc-shaped detector forms an arced inspection channel. In FIG. 3, the groove-shaped detector forms a groove-shaped inspection channel. In FIG. 4, the L-shaped detector forms an L-shaped inspection channel.

In embodiments of the arced inspection channel shown in FIG. 2, due to the characteristics of the arc shape, the path of each ray emitted from the radiation source 10 may be identical, such that the system performance is substantially uniform. This ensures that the imaging performance is substantially uniform across different detector pixels. Accordingly, the imaging system has a relatively simple structure and provides excellent imaging performance.

Furthermore, in the case of the arced inspection channel, since there is no issue of the radiation beam being incident obliquely on the gratings and the detector, i.e., the angle of the oblique incidence is very small or negligible, the extension direction of the periodic substructure of the grating (e.g., the extension direction of the grating lines) does not have a significant impact on the imaging effect. Therefore, no special requirements are imposed on the extension direction of the periodic substructure of the grating. For example, the grating lines may be arranged parallel to the extension direction of the inspection channel 30 (i.e., the Z direction), or perpendicular to the extension direction of the inspection channel 30 (i.e., the Z direction). The extension direction of the periodic substructure of the grating (e.g., the extension direction of the grating lines) may form an arbitrary angle with the extension direction of the inspection channel 30 (i.e., the Z direction).

By way of example, as shown in FIG. 2, the radiation source 10 may be arranged centrally with respect to each grating and the detector 20, and the curvature of the gratings and the detector may be configured such that a distance from the radiation source 10 to each point on each grating and the detector 20 is identical. This ensures uniform imaging performance for each point of the detector, thereby optimizing image quality.

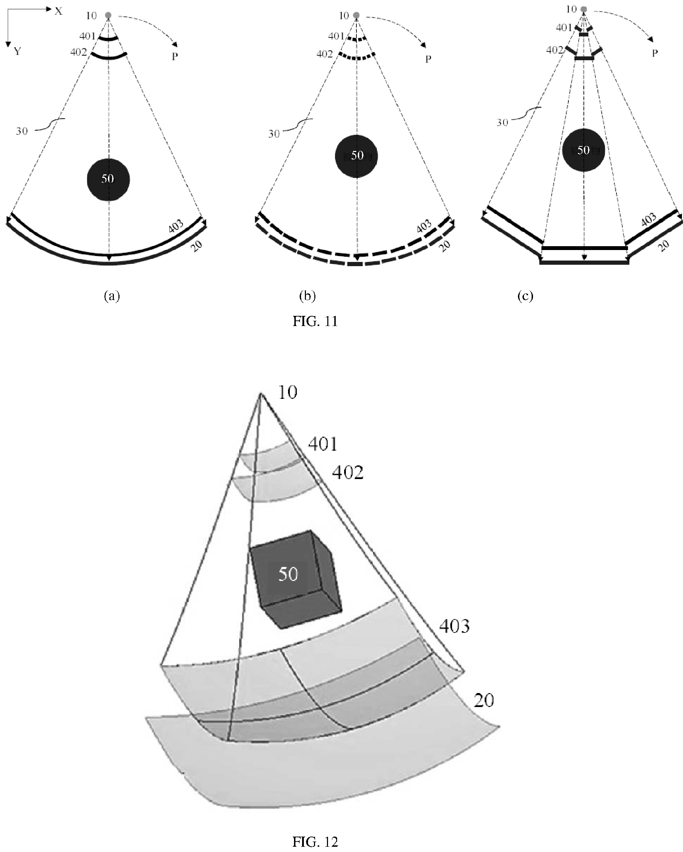

In an embodiment, the gratings 401 to 403 and the detector 20 in FIG. 2 may be formed as integral structures. By way of example, the gratings 401 to 403 may be fabricated using spherical gratings so as to achieve integral arc surface structures of the gratings. By way of example, FIG. 12 shows a perspective view of a radiation imaging apparatus 100 according to an embodiment of the present disclosure in which the gratings have arc surface structures.

In another embodiment, the gratings 401 to 403 and the detector 20 in FIG. 2 may be formed as separated structures. FIG. 5 shows a schematic diagram of another embodiment of the radiation imaging apparatus 100 according to embodiments of the present disclosure. As shown in FIG. 5, the gratings 401 to 403 and the detector 20 each may be formed by a plurality of surface segments. Herein, a "surface segment" may refer to a sub-portion of a grating, such that all sub-portions collectively constitute the entire grating. For example, a plurality of surface segments may be arranged sequentially in the X direction to form a grating. For another example, each surface segment of a single grating may have the same size.

The third grating 403 will be described below as an example. The third grating 403 may include a plurality of surface segments D. These surface segments D may be arranged in the X direction to form an arc surface, thereby forming the third grating 403 having an arc surface structure. Similarly, the first grating 401, the second grating 402, and the detector 20 may each be formed by respective surface segments, and the sizes of the surface segments may vary as desired. In the case of a separated structure, incomplete imaging may occur at the junctions between the surface segments. Accordingly, image difference processing may be performed in subsequent image processing.

By way of example, the plurality of surface segments D may be arranged to abut against each other, thereby forming a substantially continuous arc surface structure, as shown in FIG. 2. As another example, the plurality of surface segments D may be arranged to be spaced apart from each other, as shown in FIG. 5.

In embodiments shown in FIG. 3 and FIG. 4, the groove-shaped inspection channel and the L-shaped inspection channel have characteristics of a rectangular shape. In the present disclosure, a rectangular configuration refers to a shape with a right-angled corner and does not necessarily imply a complete rectangular shape. Therefore, the inspection channel having a rectangular configuration may be achieved using planar gratings. Compared with the arced channel, the gratings in the rectangular channel do not need to be bent, thereby reducing process requirements and cost. This is advantageous for reducing the overall size of the system and is more suitable for field application scenarios.

As shown in FIG. 3 and FIG. 4, the gratings and the detector may each include a first portion extending in the X direction and a second portion extending in the Y direction. The third grating 403 will be described below as an example. In the case of a groove-shaped surface structure, the third grating 403 includes a first portion 4031 extending in the X direction, and two second portions 4032 and 4033 extending in the Y direction and arranged on opposite sides of the first portion 4031. The first portion 4031 and the two second portions 4032 and 4033 collectively constitute the third grating 403, and the second portions 4032 and 4033 are each perpendicular to the first portion 4031, thereby forming a groove-shaped surface structure having rectangular characteristics. For another example, in the case of an L-shaped surface structure, the third grating 403 includes a first portion 4031 extending in the X direction, and a second portion 4032 extending in the Y direction and arranged on a side of the first portion 4031. The first portion 4031 and the second portion 4032 constitute the third grating 403 and are perpendicular to each other, thereby forming an L-shaped surface structure having rectangular characteristics.

In the case of a rectangular channel, because oblique incidence of radiation on the gratings is more likely to occur, the extension direction of each substructure of the grating should not be parallel to the extension direction of the inspection channel 30 (i.e., the Z direction), since such an arrangement may maximize the impact of oblique incidence. In other words, in the case of a grating having a groove-shaped surface structure or an L-shaped surface structure, the extension direction of each substructure needs to form a non-zero angle with the extension direction of the inspection channel 30 (i.e., the Z direction) to reduce the impact of oblique incidence. Preferably, as shown in FIG. 6, the extension direction of each substructure (e.g., each grating line 400S) is perpendicular to the extension direction of the inspection channel 30 (i.e., the Z direction). However, other angles may also be employed and may achieve similarly favorable results. That is, each grating line 400S may extend in the Y direction perpendicular to the Z direction to minimize the impact of oblique incidence, thereby ensuring imaging performance.

Although the groove-shaped channel of FIG. 3 differs from the L-shaped channel of FIG. 4, the difference primarily lies in that a portion is omitted in the gratings and the detector of FIG. 4 compared with the embodiment of FIG. 3. For example, taking the third grating 403 as an example, the embodiment of FIG. 4 omits the third portion 4033 that is present in the embodiment of FIG. 3. With respect to other aspects such as grating arrangements and imaging principles, the same content applies to the embodiments of FIG. 3 and FIG. 4. Accordingly, the embodiment of the L-shaped channel of FIG. 4 will be described below as an example. It may be understood that the following description with reference to FIG. 4 is equally applicable to the groove-shaped embodiment of FIG. 3.

Furthermore, by configuring the gratings and the detector in FIG. 4 to include only two portions, material consumption may be reduced and economic costs may be lowered. It may further be noted that the arrangement position of the radiation source 10 may vary according to the particular configuration of the groove-shaped structure and the L-shaped structure. However, the position may be adjusted as desired according to practical requirements, for example, as long as the desired imaging effect may be achieved.

As shown in FIG. 4, each of the first grating 401, the second grating 402, the third grating 403, and the detector 20 may include two portions. For example, the first grating 401 may include a first portion 4011 and a second portion 4012. The second grating 402 may include a first portion 4021 and a second portion 4022. The third grating 403 may include a first portion 4031 and a second portion 4032. The detector 20 may include a first portion 201 and a second portion 202. The corresponding first portion and second portion may be arranged at an angle relative to each other, for example, perpendicular to each other to form an L-shaped channel. Corresponding portions of the first grating 401, the second grating 402, the third grating 403, and the detector 20 are arranged parallel to each other.

In an embodiment, the gratings 401 to 403 and the detector 20 shown in FIG. 4 may be formed as integral structures. For example, each of the gratings and the detector 20 may be integrally formed. In this way, installation process of the gratings and the detector may be simplified. Furthermore, due to the integrity of the integrally formed structure, missing angular data when imaging may be avoided, thereby eliminating the need for subsequent interpolation processing. As described above, similarly, each of the gratings 401 to 403 and the detector 20 shown in FIG. 3 may also be formed integrally.

In another embodiment, for each of the gratings 401 to 403 and the detector 20 shown in FIG. 4, the two portions may be formed separately rather than integrally. For example, the first portion 4031 and the second portion 4032 of the third grating 403 may be fabricated separately and then arranged together to form the third grating 403. In such embodiments, for each of the gratings or the detector, an individual portion may be referred to as a "surface segment". Thus, each of the gratings and the detector may be understood as including a plurality of surface segments (e.g., two surface segments), which are arranged at an angle relative to each other (e.g., perpendicular to each other) to form an L-shaped surface structure. Such a configuration facilitates fabrication of the gratings. For example, planar gratings may be used to separately fabricate respective portions of a grating, which may then form a grating device through a simple arrangement. Furthermore, parameters of each grating portion or detector portion may be modified easily, thereby improving arrangement flexibility. Since each portion is fabricated separately, geometric parameters of the gratings, imaging parameters of the detector, and the like may be independently configured for each portion according to the imaging characteristics, thereby achieving optimal imaging performance in each portion.

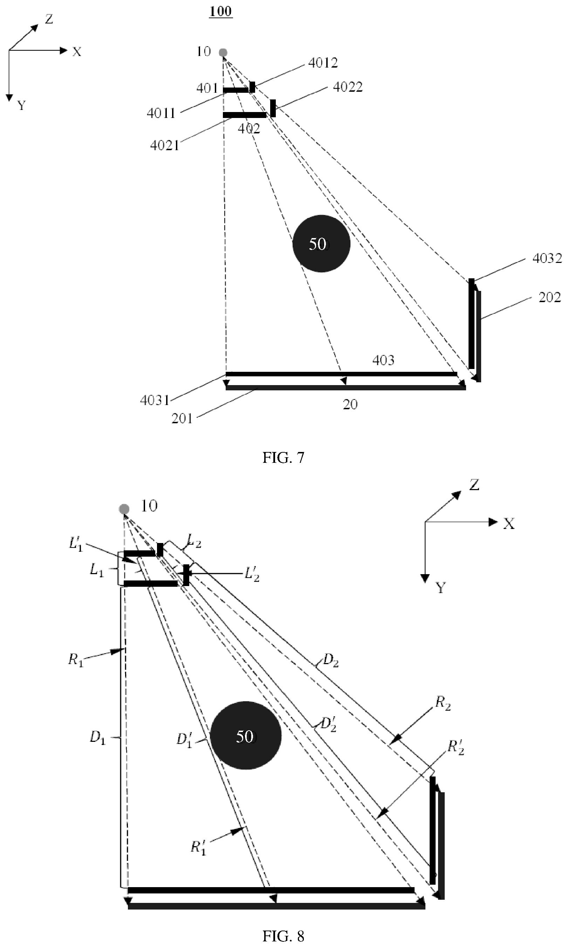

Furthermore, in such a configuration, corresponding portions of the gratings and the detector may form separate imaging regions. By way of example, as shown in FIG. 7, the first grating 401, the second grating 402, the third grating 403, and the detector 20 may include their respective second portions 4012, 4022, 4032, and 202. These second portions 4012, 4022, 4032, and 202 are arranged parallel to each other along a propagation path of the radiation beam, thereby forming a corresponding imaging region, such as a second imaging region of the radiation imaging apparatus 100. Similarly, the radiation imaging apparatus 100 may further include a first imaging region formed by the respective first portions 4011, 4021, 4031, and 201 of the gratings and the detector. Configuration parameters (e.g., geometric parameters and grating parameters) of the first imaging region and the second imaging region may be identical to or different from each other according to practical requirements. For example, in subsequent image processing, images obtained from the respective imaging regions of the radiation imaging apparatus 100 may be fused to obtain a final inspection image of the object under inspection 50.

Further, as shown in FIG. 8, in each imaging region, a ratio of spacing between adjacent gratings among the plurality of gratings is identical for each radiation ray. For example, as shown in FIG. 8, in the first imaging region formed by the first portions, a ratio L₁/D₁ of a spacing L₁ between the first portion 4011 of the first grating 401 and the first portion 4012 of the second grating 402 to a spacing D₁ between the first portion 4012 of the second grating 402 and the first portion 4031 of the third grating 403, which corresponds to a ray R₁, is equal to a ratio L₁'/D₁' corresponding to any other ray R₁'. Similarly, in the second imaging region formed by the second portions, a ratio L₂/D₂ of a spacing L₂ between the second portion 4012 of the first grating 401 and the second portion 4022 of the second grating 402 to a spacing D₂ between the second portion 4022 of the second grating 402 and the second portion 4032 of the third grating 403, which corresponds to a ray R₂, is equal to a ratio L₂'/D₂' corresponding to any other ray R₂'. In this way, in each imaging region, corresponding segments of the plurality of gratings are parallel to each other and produce a consistent effect on each ray. Accordingly, each imaging region may constitute an independent and complete imaging sub-system capable of performing imaging operations separately. This ensures imaging performance while simplifying grating fabrication, and also enhances arrangement flexibility. That is, the various parameters of each imaging sub-system formed by a corresponding imaging region may be independently designed so as to optimize the performance of each imaging sub-system.

In addition, in such a configuration, incomplete imaging may occur at the junctions between different portions or surface segments. Thus, image difference processing may be performed in subsequent image processing.

By way of example, in each of the gratings 401 to 403 and the detector 20, the plurality of surface segments or portions may be arranged to abut against each other. As another example, in each of the gratings 401 to 403 and the detector 20, the plurality of surface segments or portions may be arranged to be spaced apart from each other by a predetermined distance. For example, as shown in FIG. 7, the two surface segments or portions 201 and 202 of the detector 20 may be arranged to be spaced apart from each other by a certain distance. Alternatively, the two surface segments or portions 201 and 202 may be arranged to abut against each other. Thus, through the separated configuration of the gratings and the detector, the arrangement flexibility may be further improved. Furthermore, sufficient adjustment space may be provided for the gratings, such that the relative position of the grating (e.g., the third grating) may be adjusted more easily according to imaging effects so as to optimize image quality.

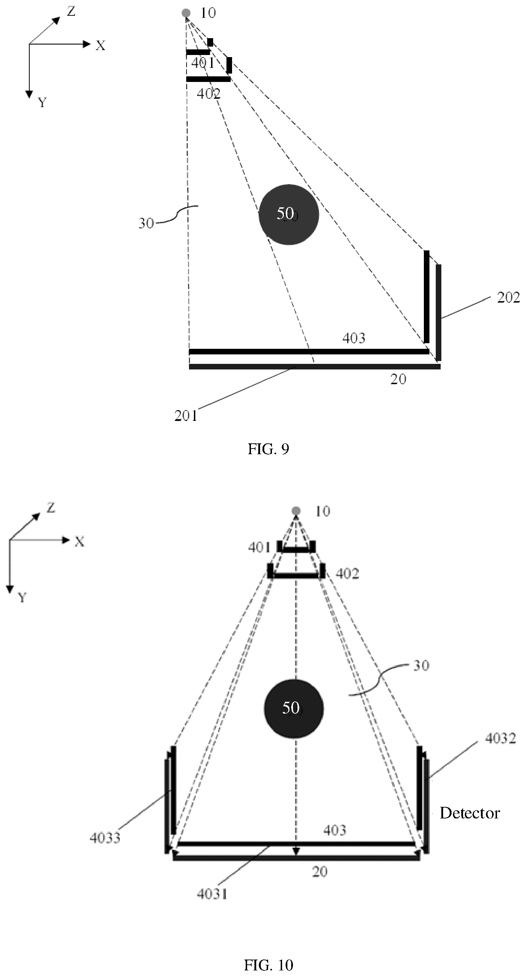

As a further example, as shown in FIG. 9, in each of the gratings 401 to 403 and the detector 20, the plurality of surface segments or portions may be arranged in a staggered or overlapping manner. For example, the second portion 202 of the detector 20 may be arranged on a side portion of the first portion 201 in the X direction and may overlap with the first portion 201 in the Y direction. In one case, the first portion 201 and the second portion 202 may be staggered relative to each other while being spaced apart from each other by a predetermined distance. Alternatively, the first portion 201 and the second portion 202 may be staggered relative to each other while abutting against each other. In this way, the staggered or overlapping arrangement may avoid the problem of missing angular data at the junctions between different portions or surface segments, thereby eliminating the need for subsequent interpolation processing and reducing computational complexity.

Similarly, as shown in FIG. 10, in the embodiment of the groove-shaped channel, each of the gratings and the detector may also be formed in a separated configuration using a plurality of portions or surface segments. Furthermore, the content described above with reference to the embodiment of the L-shaped channel, such as the arrangement of gratings and/or detector, may apply to the embodiment of the groove-shaped channel.

In an embodiment, the radiation source 10 according to embodiments of the present disclosure may be a single-energy radiation source, such that the radiation imaging apparatus 100 performs single-energy imaging. In another embodiment, the radiation source 10 according to embodiments of the present disclosure may be a dual-energy radiation source or a multi-energy radiation source, such that the radiation imaging apparatus 100 performs dual-energy imaging or multi-energy imaging. For example, two or more radiation sources (e.g., X-ray sources) emitting radiation beams having different energy spectra may be employed as the radiation source 10 to achieve dual-energy imaging or multi-energy imaging. As another example, a single radiation source may be configured to alternately emit radiation beams having two or more energy spectra to achieve dual-energy imaging or multi-energy imaging.

Embodiments of the present disclosure further relate to a CT imaging apparatus. The CT imaging apparatus may include a slip ring configured to be rotatable and the radiation imaging apparatus 100 according to embodiments of the present disclosure. The radiation imaging apparatus 100 may be arranged on the slip ring so as to rotate with the slip ring.

Parts (a) to (c) of FIG. 11 show schematic diagrams of different embodiments of a CT imaging apparatus according to embodiments of the present disclosure. Part (a) of FIG. 11 shows a CT imaging apparatus employing the radiation imaging apparatus 100 of the embodiment shown in FIG. 2. Part (b) of FIG. 11 shows a CT imaging apparatus employing the radiation imaging apparatus 100 of the embodiment shown in FIG. 5. Part (c) of FIG. 11 shows a CT imaging apparatus employing the radiation imaging apparatus 100 according to another embodiment, in which each of the plurality of gratings and the detector in the radiation imaging apparatus 100 is formed by a smaller number of surface segments having larger sizes. As shown in parts (a) to (c) of FIG. 11, the radiation imaging apparatus 100 may rotate in direction P via the slip ring. By way of example, the radiation source 10, the detector 20, the first grating 401, the second grating 402, and the third grating 403 may be arranged on the slip ring such that the inspection channel 30 passes through the slip ring.

Embodiments of the present disclosure further relate to a radiation imaging method. The method includes: irradiating an object under inspection 50 located in the inspection channel 30 with a radiation beam by using the radiation imaging apparatus 100 according to the present disclosure; and collecting signals using the detector 20 so as to generate, based on the signals detected by the detector 20, at least one of an absorption image, a phase image, or a small-angle scattering image related to at least a partial region of the object under inspection 50 in the inspection channel 30.

Embodiments of the present disclosure further relate to a CT imaging method. The method includes: irradiating an object under inspection 50 located in the inspection channel 30 with a radiation beam by using the CT imaging apparatus according to the present disclosure; and collecting signals using the detector 20 so as to generate, based on the signals detected by the detector 20, at least one of a three-dimensional absorption image, a three-dimensional phase image, or a three-dimensional small-angle scattering image related to at least a partial region of the object under inspection 50 in the inspection channel 30.

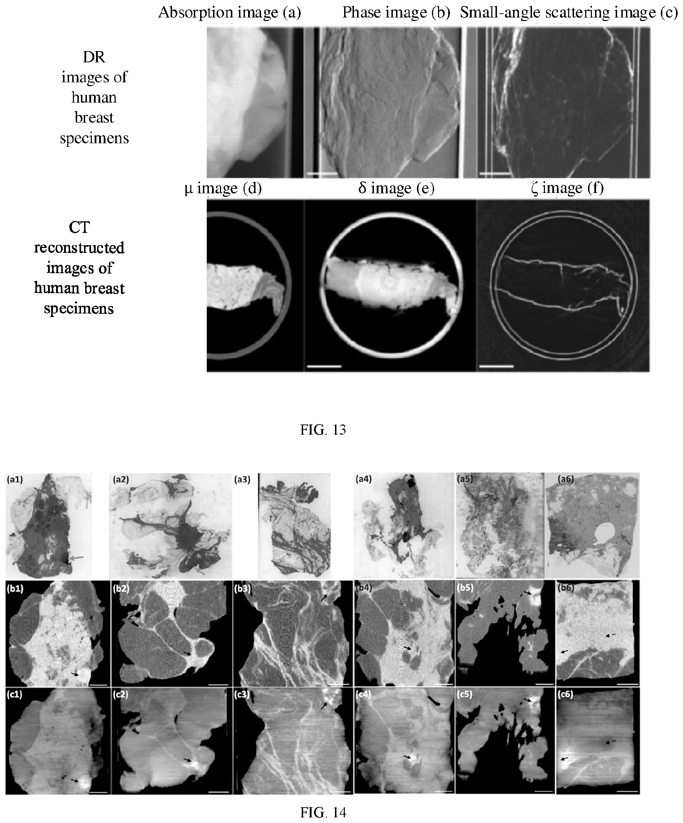

FIG. 13 shows two-dimensional images for absorption *T* (a), phase Φ (b), and small-angle scattering *D* (c), as well as corresponding XY-plane slice images of three-dimensional images for linear attenuation coefficient *µ* (d), refractive index decrement *δ* (e), and linear diffusion coefficient *ζ* (f) reconstructed using an FBP algorithm from 360 projections acquired over one rotation (corresponding to Equation (3) above). The absorption image (corresponding to the linear attenuation coefficient *µ* ) reflects attenuation characteristics of the substance. The phase image (corresponding to the refractive index decrement *δ* in the complex refractive index) provides higher contrast for weakly absorbing substances and reflects a texture information of internal and external structures of the object under inspection W. The small-angle scattering image (corresponding to the linear diffusion coefficient *ζ*) reflects the mesoscopic structural information of the substance and reflects microscopic structures such as porosity and multi-fiber structures at the micrometer/sub-micrometer scale.

FIG. 14 shows stained histological microscopic images (a1 to a6) of human breast specimens 1 to 6, as well as corresponding XZ-plane slice images of reconstructed three-dimensional images for linear attenuation coefficient *µ* (b1 to b6) and XZ-plane slice images of reconstructed three-dimensional images for refractive index decrement *δ* (c1 to c6).

In specimens 1, 2, 4, and 5, the arrows indicate tumor lesions (darkened regions); in specimen 3, the arrow indicates pectoral muscle tissue; in specimen 6, the arrow indicates a necrotic region (darkened region).

In all specimen images, fat tissues may be readily distinguished. However, in the reconstructed image with phase information, a tumor region may be more clearly distinguished from surrounding fibroglandular tissues, and a necrotic region may also be distinguished.

It may be understood that the various forms of processes shown above may be used, with steps reordered, added, or deleted. For example, the steps described herein may be performed in parallel, sequentially, or in a different order, as long as the desired results of the technical solutions provided by the present disclosure may be achieved, and no limitation is imposed herein.

In the technical solutions of the present disclosure, the collection, storage, use, processing, transmission, provision, disclosure, etc., of user personal information involved all comply with relevant laws and regulations and do not violate public order and good morals.

The foregoing specific embodiments are not intended to limit the scope of protection of the present disclosure. Those skilled in the art will appreciate that various modifications, combinations, sub-combinations, and substitutions may be made to the disclosed embodiments or examples according to design requirements and other factors. Any modification, equivalent substitution, and improvement within the spirit and principle of the present disclosure shall fall within the scope of protection of the present disclosure.

## Claims

1. A radiation imaging apparatus, comprising:
a radiation source configured to emit a radiation beam;
a detector configured to receive the radiation beam from the radiation source, wherein the radiation source and the detector define an inspection channel; and
one or more gratings arranged in the inspection channel and between the radiation source and the detector;
wherein the one or more gratings have one of an arc surface structure, a groove-shaped surface structure, or an L-shaped surface structure, the detector is in one of an arc shape, a groove shape, or an L shape to extend parallel to the arc surface structure, the groove-shaped surface structure, or the L-shaped surface structure of the one or more gratings, and the arc surface, the groove-shaped surface, or the L-shaped surface is arranged to face the radiation source to receive the radiation beam emitted by the radiation source.

2. The radiation imaging apparatus of claim 1, wherein the one or more gratings comprise periodic substructures configured to diffract rays to generate an interference fringe.

3. The radiation imaging apparatus of claim 2, wherein the one or more gratings comprise a spherical grating.

4. The radiation imaging apparatus of claim 2, wherein for the one or more gratings having the groove-shaped surface structure or the L-shaped surface structure, an extension direction of each of the periodic substructures forms a non-zero angle with an extension direction of the inspection channel.

5. The radiation imaging apparatus of claim 4, wherein the extension direction of each of the periodic substructures is perpendicular to the extension direction of the inspection channel.

6. The radiation imaging apparatus of claim 1, wherein the detector and the one or more gratings each have an integral structure.

7. The radiation imaging apparatus of claim 1, wherein the detector and the one or more gratings each comprise a plurality of surface segments forming an arc surface, or comprise a plurality of angled surface segments forming a groove-shaped surface or an L-shaped surface.

8. The radiation imaging apparatus of claim 7, wherein the plurality of surface segments of the arc surface, or the plurality of angled surface segments of the groove-shaped surface or the L-shaped surface, abut against each other or are spaced apart from each other.

9. The radiation imaging apparatus of claim 1, wherein the one or more gratings comprise:
a first grating arranged adjacent to and parallel to the radiation source and spaced apart from the radiation source by a first distance; and/or
a second grating arranged adjacent to and parallel to the radiation source and spaced apart from the radiation source by a second distance different from the first distance; or a second grating arranged adjacent to and parallel to the detector and spaced apart from the detector by a third distance; and/or
a third grating arranged adjacent to and parallel to the detector and spaced apart from the detector by a fourth distance different from the third distance.

10. The radiation imaging apparatus of claim 9, comprising the first grating, the second grating, and the third grating, wherein the first grating, the second grating, and the third grating are parallel to each other.

11. The radiation imaging apparatus of claim 1, wherein the radiation imaging apparatus is configured to generate, based on signals detected by the detector, at least one of an absorption image, a phase image, or a small-angle scattering image related to at least a partial region of an object under inspection in the inspection channel.

12. The radiation imaging apparatus of claim 1, wherein the radiation source is a dual-energy radiation source.

13. A CT imaging apparatus, comprising:
a slip ring configured to be rotatable; and
the radiation imaging apparatus of any one of claims 1 to 12, wherein the radiation imaging apparatus is arranged on the slip ring to rotate with the slip ring;
wherein the radiation source, the detector, and the one or more gratings are arranged on the slip ring, and the inspection channel passes through the slip ring.

14. A radiation imaging method, comprising:
irradiating an object under inspection located in an inspection channel with a radiation beam by using the radiation imaging apparatus of any one of claims 1 to 12; and
collecting signals using a detector so as to generate, based on the signals detected by the detector, at least one of an absorption image, a phase image, or a small-angle scattering image related to at least a partial region of the object under inspection in the inspection channel.

15. A CT imaging method, comprising:
irradiating an object under inspection located in an inspection channel with a radiation beam by using the CT imaging apparatus of claim 13; and
collecting signals using a detector so as to generate, based on the signals detected by the detector, at least one of a three-dimensional absorption image, a three-dimensional phase image, or a three-dimensional small-angle scattering image related to at least a partial region of the object under inspection in the inspection channel.
